# EUROPÄISCHE PATENTSCHRIFT

(11) **EP 1 535 967 B1**
(45) Veröffentlichungstag und Bekanntmachung des Hinweises auf die Patenterteilung: **03.08.2011**
(21) Anmeldenummer: 05000174.2
(22) Anmeldetag: 05.09.2001
(51) Int. Cl.: C09B 19/00, G01N 33/533, C07D 265/34

(54) **Verfahren zur gezielten Markierung mittels affinitätsmarkierter Detektionsmarker**
Method for selective marking using affinity tagged detection markers
Méthode de marquage sélectif à l'aide des réactifs de détection contenant un marquage d'affinité

(30) Priorität: 06.09.2000 EP 00119292; 08.12.2000 EP 00127005
(43) Veröffentlichungstag der Anmeldung: 01.06.2005
(62) Teilanmeldung aus: 01978341.4
(73) Patentinhaber: Evotec AG, 22525 Hamburg (DE)
(72) Erfinder: Fries, Joachim, 4800 Zofingen (CH); Lopez-Calle, Eloisa, 20535 Hamburg (DE); Drexhage, Karl Heinz, 57076 Siegen (DE); Winkler, Dirk, 04275 Leipzig (DE)
(74) Vertreter: Hertz, Oliver

(56) Entgegenhaltungen:
- EP-A- 0 323 152
- EP-A- 0 747 447
- WO-A-01/42505
- WO-A-90/03383
- WO-A-97/29154
- WO-A-99/07793
- DE-A- 2 411 761
- DE-A- 4 023 212
- US-A- 4 714 763
- US-A- 4 780 535
- US-A- 5 532 171
- US-A- 5 739 318
- US-A- 5 792 389
- US-A- 6 140 500

## Beschreibung

Die vorliegende Erfindung betrifft ein Verfahren zur Markierung von biologischem und nicht-biologischem Material mittels affinitätsmarkierter Detektionsmarker.

Durch herkömmliche Markierungsverfahren, insbesondere Proteinmarkierungsverfahren beispielsweise unter Verwendung von fluoreszenten Detektionsmarkern werden immer mehrere reaktive Gruppen dieser Moleküle angesprochen, so dass es durch die Markierungsreaktion zu einer statistischen Markierung kommt, d.h. einige Proteine besitzen keine, andere eine, zwei oder mehr Markierungen. In hochempfindlichen Analysetechniken kommt es durch die heterogene Analytmischung zu unerwünschten heterogenen Signalverteilungen, die die Messergebnisse verfälschen oder gar unbrauchbar machen. Darüber hinaus kommt es beispielsweise innerhalb von mehrfach markierten Proteinen durch die Wechselwirkung zwischen den einzelnen Markierungen zu einer Abschwächung des Signals.

Die Aufgabe der vorliegenden Erfindung besteht darin, ein Verfahren zur Markierung von Material bzw. Molekülen zur Verfügung zu stellen, welches die vorstehend genannten Nachteile der bekannten Markierungsverfahren vermeidet.

Die Lösung der Aufgabe erfolgt erfindungsgemäß durch das Verfahren mit den Merkmalen der Ansprüche 1 bis 8.

Prinzipell kann jeder messbare Detektionsmarker, beispielsweise auch radioaktive Detektionsmarker etc., eingesetzt werden. Bevorzugt werden Farbstoffmarker, insbesondere Fluoreszenzmarker, verwendet werden. Ganz besonders bevorzugt werden Oxazinderivate eingesetzt.

Anstelle der einfach markierten Fraktionen des zu untersuchenden Materials, kann es beispielsweise auch bevorzugt sein, zweifach, dreifach etc. markierte Fraktionen zu isolieren.

Die erfindungsgemäßen Detektionsmarker weisen zusätzlich mindestens einen Affinitätsmarker auf, der eine einfache Reinigung durch Affinitätschromatographie ermöglicht. Der Affinitätsmarker kann z. B. an eine Linkerverbindung gebunden sein.

In einer bevorzugten Ausführungsform weisen die Detektionsmarker nur einen Affinitätsmarker auf. Im Prinzip kann jeder Affinitätsmarker verwendet werden, der geeignet ist, in der nachfolgenden Affinitätschromatographie eine saubere Trennung durchzuführen. Als Beispiele können an dieser Stelle Affinitätsmarker, wie Biotin, Hexa-His oder Haptene genannt werden.

Das Verfahren zur gezielten Markierung von zu untersuchendem Material mittels affinitätsmarkierter Detektionsmarker nach Anspruch 1 wird wie folgt durchgeführt:
a) Bereitstellen einer Probe, umfassend ein zu untersuchendes Material;
b) Umsetzung des zu untersuchenden Materials mit einem oben beschriebenen, einen Affinitätsmarker enthaltenden Detektionsmarker, wobei sich ein markiertes zu untersuchendes Material bildet, das als Mischung kein, ein oder mehrere Detektionsmarker als Markierung aufweist; und
c) Auftrennung des zu untersuchenden Materals in nicht, einfach oder mehrfach markierte Fraktionen durch Affinitätschromatographie.

Aus WO 00/02052 ist die Kombination eines Affinitätsliganden mit einem sogenannten Effektor, der z.B. ein Fluorophor sein kann, sowie mit einem reaktiven Linker, der zur Anbindung eines Biomoleküls dient, unter Verwendung einer trifunktionellen Brückeneinheit bekannt. Der für das erfindungsgemäße Markierungsverfahren wesentliche Schritt der Auftrennung des markierten Materials mittels Affinitätschromatographie in nicht einfach oder mehrfach markierte Fraktionen wird in diesem Dokument weder beschrieben noch vorgeschlagen.

Nachfolgend wird beispielhaft eine derartige Affinitätsaufreinigung unter Verwendung von Oxazinderivaten als Detektionsmarker mit dem System Biotin/Avidin (Affinitätsmarker/Trägermaterial) vorgestellt.

Durch den Einsatz des unten dargestellten affinitätsmarkierten Farbstoff-Reaktiv-Moleküls kann der oben beschriebene Nachteil der bekannten Markierungstechniken umgangen werden. Dabei steht die Biotin-Einheit stellvertretend für einen beliebigen Affinitätsmarker, Oxazinderivat III für einen beliebigen Farbstoffmarker und der N-Succinimidylester für eine beliebige reaktive Gruppe. Ein solches Molekül reagiert analog den herkömmlichen Markierungsverfahren mit dem Protein, was in einer heterogenenen Mischung wie oben beschrieben resultiert. Durch eine nachfolgende Affinitätsreinigung an einem geeigneten Trägermaterial (hier monomeres Avidin) werden unmarkierte Proteine aus der Probe entfernt. Durch nachfolgende vorsichtige Elution können einfachmarkierte Proteine eluiert werden, während doppel- und höhermarkierte Proteine aufgrund der kooperativ verstärkten Bindung an das Trägermaterial gebunden bleiben. Somit kann ein homogener Analyt mit exakt einem Oxazinderivat pro Proteinmolekül erhalten werden.

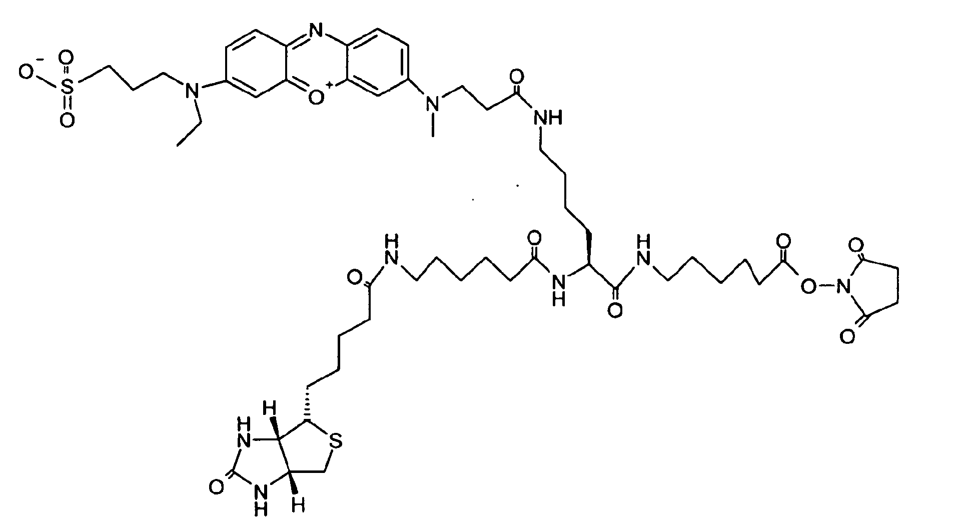

Das zu untersuchende Material kann kovalent oder nichtkovalent an das Oxazinderivat gebunden sein, wobei es allerdings bevorzugt kovalent gebunden ist. Bei dem zu untersuchenden Material handelt es sich vorzugsweise um ein biologisches Material, eine chemische Verbindung, insbesondere eine biologisch aktive Substanz und/oder einen pharmazeutischen Wirkstoff, einen synthetischen oder biologischen Mikropartikel oder eine Kombination davon.

Die chemischen Verbindungen können alle synthetischen Verbindungen umfassen, die als ein einzelnes Molekül oder Aggregat, beispielsweise als kleine organische Verbindung und auch als Oligomer oder Polymer vorliegen. Vorzugsweise handelt es sich bei den chemischen Verbindungen um biologisch aktive Substanzen und/oder pharmazeutische Wirkstoffe.

Synthetische Mikropartikel, d.h. insbesondere lösliche und suspendierbare Trägermaterialien, bezeichnen jede Art von synthetischen Mikropartikeln, die in einer Flüssigkeit, insbesondere einer wässrigen Lösung, gelöst oder suspendiert werden können. Die Durchmesser d der Mikropartikel sind gleich oder kleiner als 1 µm, bevorzugt liegen sie im Bereich von 1 nm ≤ d ≤ 1 µm, besonders bevorzugt im Bereich von 10 nm ≤ d ≤ 500 nm, ganz besonders bevorzugt im Bereich von 50 nm ≤ d ≤ 300 nm. Beispiele für die synthetischen Mikropartikel umfassen solche aus organischen Polymeren. Zum Beispiel können Polymere auf Dendrimer-Basis, bevorzugt Polyethylenglykol auf Dendrimer-Basis verwendet werden. Es können jedoch alle synthetischen Mikropartikel verwendet werden, die unter den experimentellen Bedingungen löslich oder suspendierbar sind, insbesondere Glaspartikel mit definierter Porengröße, Cellulose-, Silikagel- und andere Typen von Polystyrolpartikeln, letztere gegebenenfalls vernetzt mit Divinylbenzol und/oder gepropft mit Polyethylenglykol und/oder funktionalisiert mit Amino, Hydroxy, Carboxyl oder Halogen. Weitere Beispiele von möglichen synthetischen Mikropartikeln umfassen gepropfte CoPolymer-, Polyacrylamid-, Latex-, gegebenenfalls mit N,N'-bis-Acryloylethylendiamin gepropfte Dimethylacrylamidpartikel und polymerüberzogene Glaspartikel. Bei den biologischen Mikropartikeln handelt es sich vorzugsweise um vesikuläre Partikel oder virus-ähnliche Partikel.

Bei dem biologischen Material kann es sich beispielsweise um Nukleotide, Oligonukleotide, Zucker, Lipide, Membranen, Zellen, Zellbestandteile, DNA, RNA, Peptide, Proteine, Antikörper, Haptene oder Antigene handeln.

Die Markierung von zu untersuchenden Materialien, insbesondere biologischem Material kann sowohl in flüssiger als auch in Verbindung mit festen Phasen erfolgen. Dabei ist sowohl die Verwendung von aktivierten als auch aktivierbaren Oxazinderivaten möglich. Beispielsweise erfolgt die Aktivierung der aktivierbaren Oxazinderivate, z.B. der Carboxyderivate, in-situ mit Aktivierungsreagenzien, wie z.B. Benzotriazol-1-yl-oxy-tris-pyrrolidino-phosphonium Hexafluorophosphat (PyBOB). Vorzugsweise wird eine solche Aktivierung bei Reaktionen an der festen Phase durchgeführt.

Das zu untersuchende Material ist vorzugsweise ein biologisches Material, eine chemische Verbindung, insbesondere eine biologisch aktive Substanz und/oder ein pharmazeutischer Wirkstoff, ein synthetischer oder biologischer Mikropartikel oder eine Kombination davon. Die einzelnen Materialien sind bereits oben beschrieben worden.

Das erfindungsgemäße Markierungsverfahren eignet sich hervorragend für chemische oder biotechnische Untersuchungen. Dazu zählen: die Identifizierung und Charakterisierung von biologischem Material oder chemischen Verbindungen, Suche nach biologisch aktiven Substanzen und/oder pharmazeutischen Wirkstoffen, die Identifizierung von Analyten in diagnostischen und/oder therapeutischen Verfahren, die Genomanalyse (z. B. SNP-Analysen) oder die Reinigung und Konzentrierung von Substraten.

Die genannten Untersuchungen werden bevorzugt mittels Laserlichtanalytik und Fluoreszenzdetektion durchgeführt. Die dabei angewandten Messverfahren schließen praktisch alle Verfahren ein, mit denen der Detektionsmarker gemessen werden kann. Zu diesen Verfahren zählen die Spektrometrie, Mehrphotonenanregung, insbesondere Zwei-Photonen-Anregung, Laser-Scanning-Mikroskopie, Nahfeldspektroskopie, Photonenverteilungsanalysen, insbesondere FIDA und 2-D-FIDA, Fluoreszenz-Lebensdauer-Analyse und Fluoreszenz-Polarisationsanalyse. Ganz besonders bevorzugt werden die Messungen unter Verwendung eines konfokalen Mikroskops oder anderen konfokalen Optiken durchgeführt.

Die Auswertung der Daten erfolgt schließlich vorzugsweise durch Autokorrelationsanalyse und /oder Kreuzkorrelationsanalyse.

Bei der 2-D-FIDA und Kreuz-Korrelationsanalyse wird ein Messvolumen einer Probe mit zwei Wellenlängen simultan bestrahlt und die Information über eine zeitliche Koinzidenzanalyse von zwei Farbstoffspezies, wie die Oxazinderivate in Kombination mit grün anregbaren Fluoreszenzfarbstoffen, erhalten.

Ähnlich ist der Einsatz in Fluoreszenz-Resonanz-Energie-Transfer-Experimenten (FRET) von großem Nutzen, wobei die Detektionsmarker mit einer Reihe von bekannten Xanthenen als Paar für den Energie-Transfer verwendet werden.

Die Detektionsmarker können ebenfalls sehr gut in FISH-(Fluorescence-in-situ-hybridization) oder PCR-Verfahren (Polymerase-chain reaction) verwendet werden.

Die erfindungsgemäß verwendbaren Oxazinfarbstoffe sind synthetisch einfach zugänglich, was sie von anderen wesentlich schwerer herstellbaren Verbindungen des Standes der Technik unterscheidet. Diese Farbstoffe, insbesondere die in EP 1 317 511 beschriebenen Derivate II, III, IV oder V, weisen alle eine sehr kompakte molekulare Struktur auf, so dass die Wechselwirkung mit dem zu untersuchenden Material als äußerst gering einzuschätzen ist. Insbesondere werden die biologischen Eigenschaften von biologischem Material nicht verändert. Die in der Regel neutrale Gesamtladung minimiert die elektrostatische Wechselwirkung zwischen dem Farbstoff und der markierten Komponente. Diese Fluoreszenzfarbstoffe weisen eine hohe Reaktivität gegenüber den zu untersuchenden Materialien, insbesondere Aminen, eine hohe Fluoreszenzquantenausbeute, eine hohe Lösungsmittelbeständigkeit und pH-Beständigkeit, eine hohe chemische Stabilität und eine NIR-Anregbarkeit auf. Diese Farbstoffe sind durch eine geringe Anregungsenergie und somit einer geringen Photozerstörung gekennzeichnet. Die besonderen Anregungs- und Emissionswellenlängen dieser Farbstoffe ermöglichen die Verwendung von kommerziell erhältlichen Filtersystemen. Vorteilhaft ist weiterhin die geringe Hintergrundfluoreszenz (Autofluoreszenz) von chemischen und biologischen Materialien bei roter Anregung gegenüber energiereicheren Anregungwellenlängen. Diese Farbstoffe sind somit sehr gut im roten Spetralbereich einsetzbar. Die hohe chemische Stabilität ist insbesondere unter Bedingungen der Festphasensynthese von Bedeutung. Die Farbstoffe zeigen eine geringe Adsorptions- oder unspezifische Bindungstendenz und sind sehr gut wasserlöslich. Sie sind somit ideal für chemische und biotechnische Untersuchungen in wässrigen Lösungen, DMSO, DMF oder Alkoholen verwendbar.

Die nachfolgenden Beispiele erläutern die Herstellung eines in der vorliegenden Erfindung verwendbaren affinitätsmarkierten Detektionsmarkers.

### Beispiel 1

Einführung eines Linkers in Oxazinderivat (III)

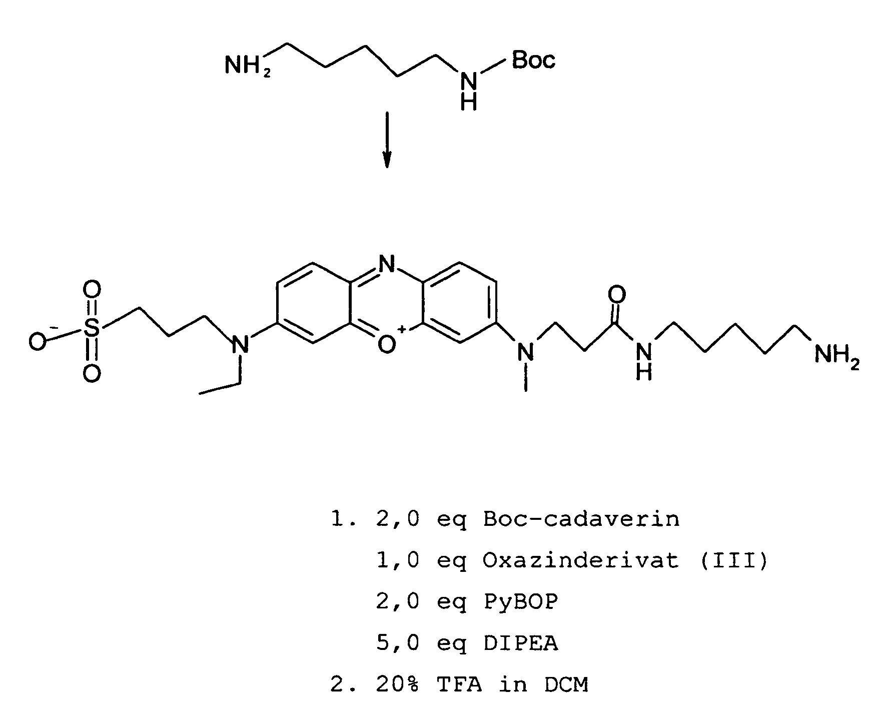

2 µmol [(2-Carboxy-ethyl)-methyl-amino]-[ethyl-(3-sulfopropyl)-amino]-phenoxazin-5-ylium (Oxazinderivat III) werden in 200 µl N,N-Dimethylformamid gelöst und eine Lösung von 4µmol mono-t-Butoxycarbonyl-1,5-diaminopentan (Boc-cadaverin) in 50 µl N,N-Dimethylformamid, eine Lösung von 4 µmol Benzotriazol-1-yl-oxy-tris-pyrrolidino-phosphonium Hexafluorophosphat (PyBOP) in 50 µl N,N-Dimethylformamid sowie 10 µmol N-Ethyl-diisopropyl-amin (DIPEA) zugegeben. Diese Lösung wird 16 Stunden bei Raumtemperatur geschüttelt und dann zur Trockne eingeengt. Der Rückstand wird mit 300 µl einer Lösung von 20 % Trifluoressigsäure (TFA) in Dichlormethan (DCM) versetzt und 30 Minuten bei Raumtemperatur geschüttelt. Die Lösung wird zur Trockne eingeengt und das Rohprodukt säulenchromatographisch über HPLC aufgereinigt.

### Beispiel 2

Einführung eines Linkers in Oxazinderivat (III) 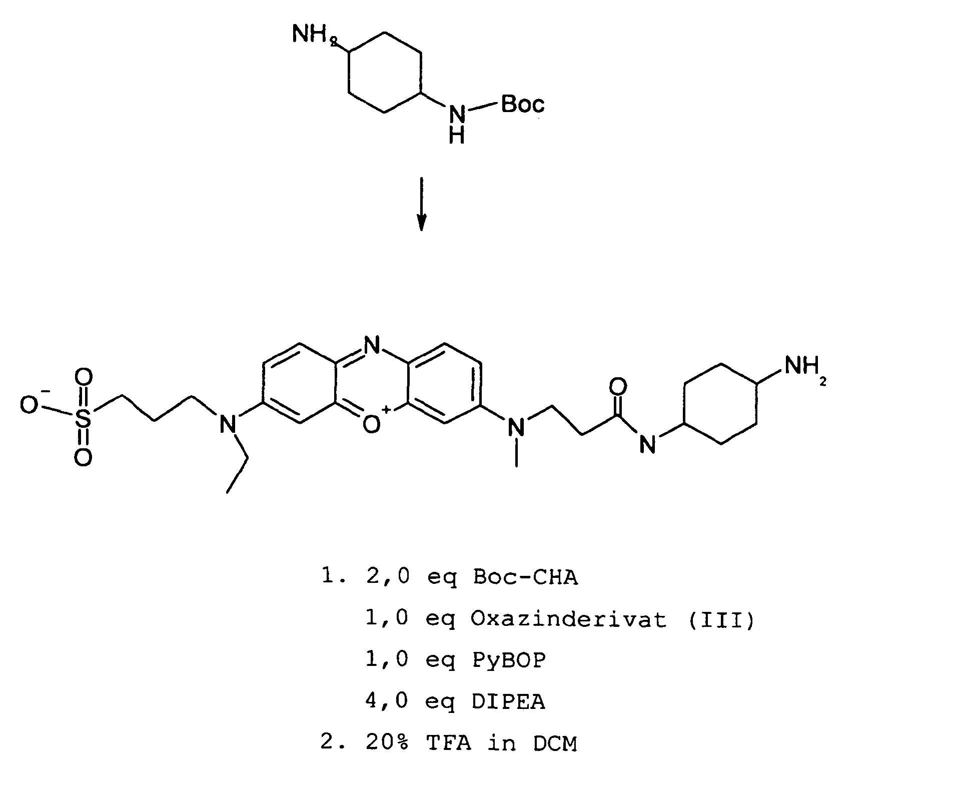 2 µmol [(2-Carboxy-ethyl)-methyl-amino]-[ethyl-(3-sulfopropyl)-amino]-phenoxazin-5-ylium(Oxazinderivat III) werden in 200 µl N,N-Dimethylformamid gelöst und eine Lösung von 4 µmol mono-t-Butoxycarbonyl-trans-1,4-diaminocyclohexan (Boc-CHA) in 50 µl N,N-Dimethylformamid, eine Lösung von 2 µmol Benzotriazol-1-yl-oxy-tris-pyrrolidino-phosphonium Hexafluorophosphat (PyBOP) in 50 µl N,N-Dimethylformamid sowie 8 µmol N-Ethyl-diisopropyl-amin (DIPEA) zugegeben. Diese Lösung wird 16 Stunden bei Raumtemperatur geschüttelt und dann zur Trockne eingeengt. Der Rückstand wird mit 300 µl einer Lösung von 20 % Trifluoressigsäure (TFA) in Dichlormethan (DCM) versetzt und 30 Minuten bei Raumtemperatur geschüttelt. Die Lösung wird zur Trockne eingeengt und das Rohprodukt säulenchromatographisch über HPLC aufgereinigt.

### Beispiel 3

### Affinitätsmarkierung

1 mmol N-ε-(9-Fluorenylmethoxycarbonyl)-aminohexansäure wurden in 1200 µl Dichlormethan und 300 µl N,N-Dimethylformamid (DMF) gelöst und auf 0°C abgekühlt. 500 µmol N,N'-Diisopropylcarbodiimid wurden in 80 µl N,N-Dimethylformamid gelöst und zur Lösung der Aminosäure hinzugegeben. Die Reaktionslösung wurde 30 Minuten bei 0°C stehengelassen und anschließend das Dichlormethan im Vakuum entfernt. Der Rückstand wurde mit 1000 µl N,N-Dimethylformamid aufgenommen, wobei eine klare Lösung erhalten wurde. Diese Lösung wurde zu 100 µmol Wang Harz (Wang Resin) in einer 5 ml Spritze mit Fritte gegeben. Zu der Suspension wurden 10 µmol Dimethylaminopyridin in 25 µl N,N-Dimethylformamid gegeben, die Mischung wurde über Nacht stehengelassen.

Das Harz wurde anschließend sechsmal mit 2 ml N,N-Dimethylformamid und dreimal mit 2 ml Dichlormethan und sechsmal mit 2 ml *t*-Butylmethylether gewaschen, getrocknet und zur Bestimmung der Beladung ausgewogen. Die bestimmte Beladung des Harzes betrug 0.8 mmol/g Harz. Zur weiteren Beladung wurde zunächst die 9-Fluorenylmethoxycarbonyl-Schutzgruppe (FMOC) mittels zweimaliger Behandlung für 5 bzw. 15 Minuten mit einer Lösung von 20 % Piperidin in N,N-Dimethylformamid abgespalten. Das Harz wurde danach sechsmal mit 2 ml N,N-Dimethylformamid gewaschen. 1 mmol N-α-(9-Fluorenylmethoxycarbonyl)-ε-(4-methyltrityl)-L-lysin, gelöst in 120 µl N,N-Dimethylformamid sowie 1 mmol 1-[Bis(dimethylamino)-methyliumyl]-1H-1,2,3-triazolo[4,5-*b*]pyridin-3-oxid hexafluorophosphat gelöst in 120 µl N,N-Dimethylformamid wurden zusammen mit 2 mmol N,N-Diisopropylethylamin zum Harz gegeben. Die Reaktionslösung wurde nach 30 Minuten abgezogen und der Vorgang der Beladung unter identischen Bedingungen nochmals wiederholt. Nach sechsmaligem Waschen des Harzes mit je 2 ml N,N-Dimethylformamid wurde die 9-Fluorenylmethoxycarbonyl Schutzgruppe mittels zweimaliger Behandlung für 5 bzw. 15 Minuten mit einer Lösung von 20 % Piperidin in N,N-Dimethylformamid abgespalten. Das Harz wurde danach wiederum sechsmal mit 2 ml N,N-Dimethylformamid gewaschen. Anschließend erfolgte die Umsetzung mit 250 µmol Biotinoylaminohexanoyl-N-hydroxysuccinimidylester in 300 µl N,N-Dimethylformamid. Nach 10 stündiger Reaktion wurde die Reaktionslösung abgesaugt und das Harz sechsmal mit 2 ml N,N-Dimethylformamid und dreimal mit 2 ml Dichlormethan und sechsmal mit 2 ml *t*-Butylmethylether gewaschen und getrocknet.

Zur Beladung mit dem Fluoreszenzfarbstoff (dye) wurden 10 µmol des Harzes abgenommen und in eine 5 ml Spritze überführt. Nachdem das Harz in 2 ml Dichlormethan vorgequollen wurde, erfolgte die Abspaltung der 4-Methyltrityl-Schutzgruppe (MTT) mittels fünfmaliger jeweils dreiminütiger Behandlung mit je 2 ml 30 % Hexafluorisopropanol (HFIP) in Dichlormethan. Danach wurde das Harz sechsmal mit 2 ml Dichlormethan und dreimal mit 2 ml N,N-Dimethylformamid gewaschen. Anschließend erfolgte die Umsetzung mit 25 µmol [(2-Carboxy-ethyl)-methyl-amino]-ethyl-(3-sulfo-propyl)-amino]-phenoxin-5-ylium-succinimidylester in 300 µl N,N-Dimethylformamid für drei Stunden bei Raumtemperatur. Nach erfolgter Reaktion wurde das Harz sechsmal mit 2 ml N,N-Dimethylformamid, sechsmal mit 2 ml Dichlormethan, sechsmal mit 2 ml Methanol und sechsmal mit 2 ml *t*-Butylmethylether gewaschen und getrocknet. Das Produkt wurde nunmehr mittels einer Mischung von 95 % Trifluoressigsäure (TFA), 2,5 % Wasser und 2,5 % Triispropylsilan (TIS) vom Harz abgespalten. Nach zweistündiger Reaktionszeit wurde die Lösung vom Harz abfiltriert und im Vakuum eingeengt. Das als Rückstand verbleibende Rohprodukt wurde anschließend in Methanol aufgenommen und chromatographisch aufgereinigt.

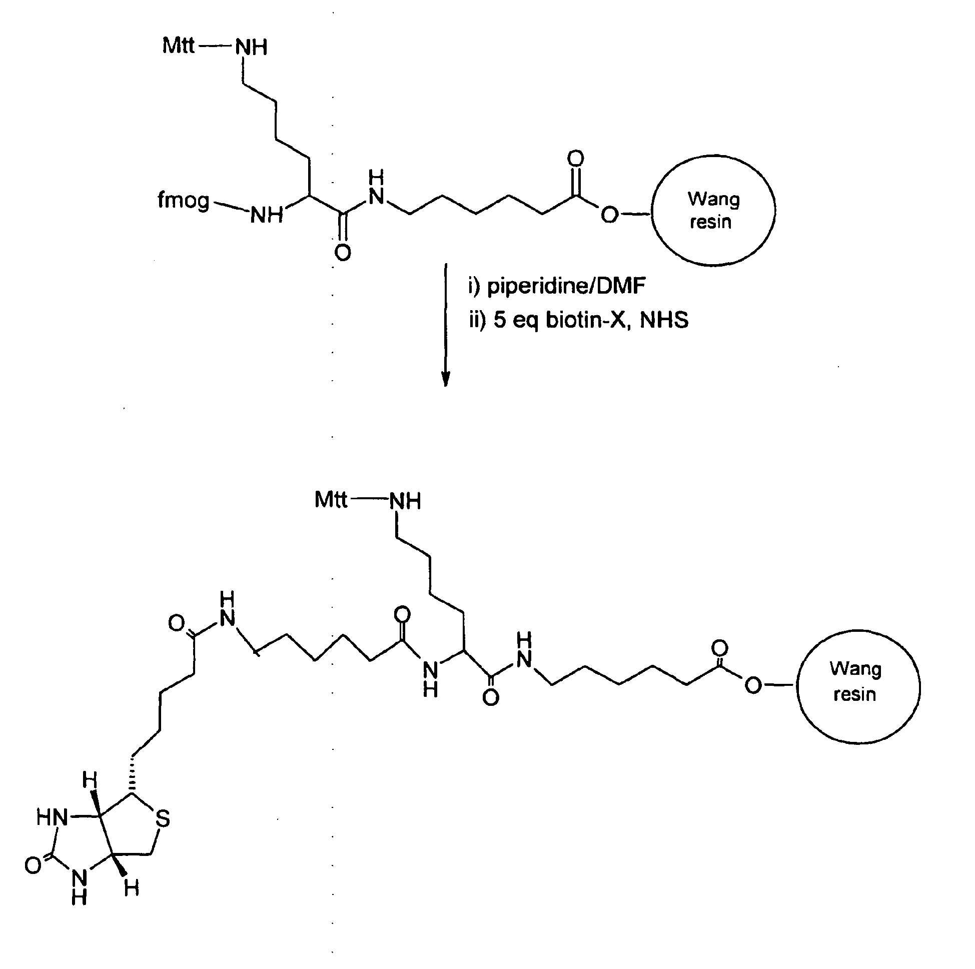

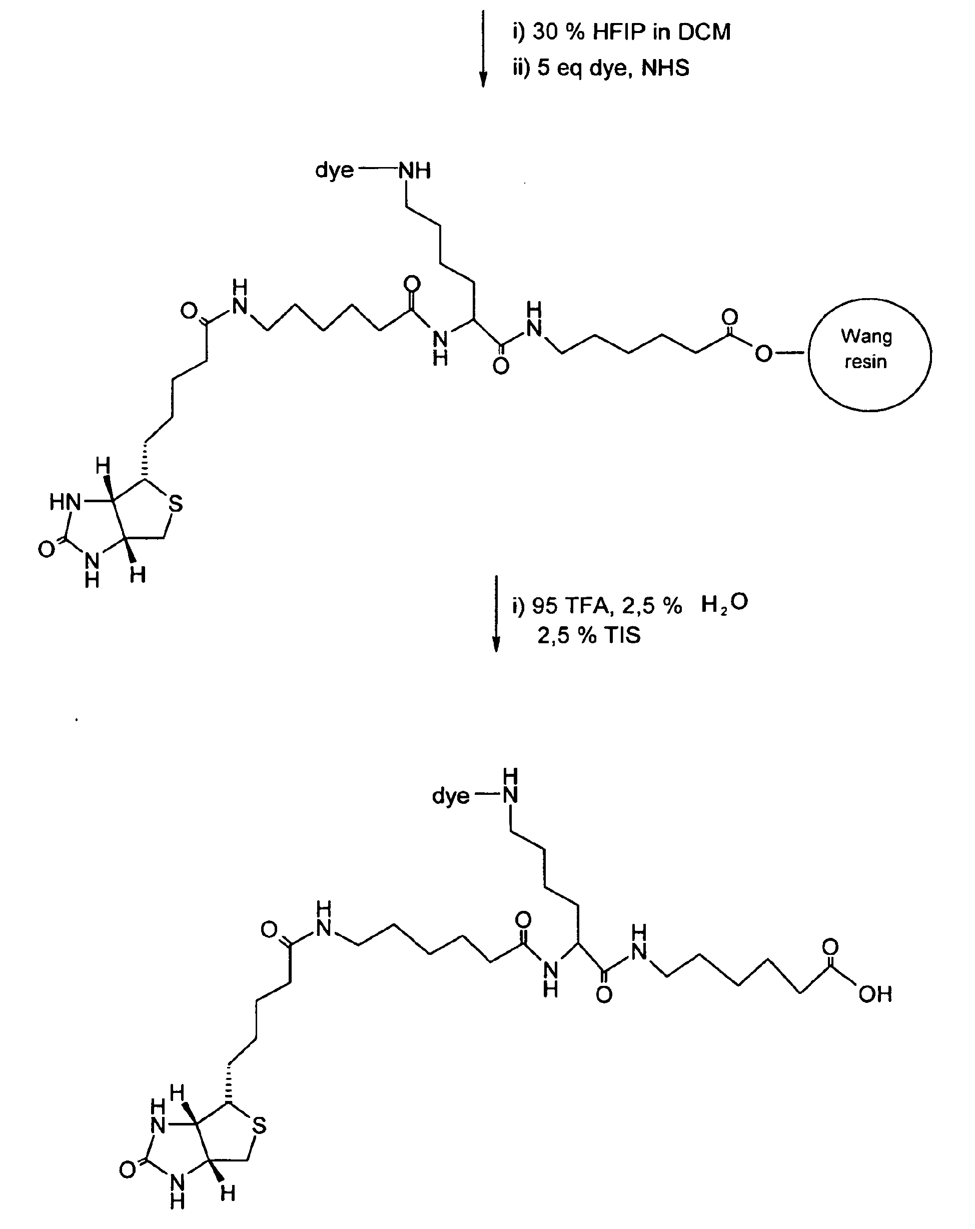

## Patentansprüche

1. Verfahren zur gezielten Markierung von zu untersuchendem Material mittels affinitätsmarkierter Detektionsmarker, umfassend die Schritte:
a) Bereitstellen einer Probe, umfassend ein zu untersuchendes Material;
b) Umsetzung des zu untersuchenden Materials mit einem einen Affinitätsmarker enthaltenden messbaren Detektionsmarker, wobei sich ein markiertes zu untersuchendes Material bildet, das als Mischung keinen, einen oder mehrere Detektionsmarker aufweist; und
c) Auftrennung des zu untersuchenden Materials in nicht, einfach oder mehrfach markierte Fraktionen durch Affinitätschromatographie.

2. Verfahren nach Anspruch 1, **dadurch gekennzeichnet, dass** als Detektionsmarker radioaktive Detektionsmarker oder Farbstoffmarker eingesetzt werden.

3. Verfahren nach Anspruch 2, **dadurch gekennzeichnet, dass** es sich bei den Farbstoffmarkern um Fluoreszenzmarker handelt.

4. Verfahren nach einem der Ansprüche 1-3, **dadurch gekennzeichnet, dass** es sich bei dem zu untersuchenden Material um
i) ein biologisches Material,
ii) eine chemische Verbindung,
iii) einen synthetischen oder biologischen Mikropartikel oder
iv) eine Kombination davon handelt.

5. Verfahren nach Anspruch 4, **dadurch gekennzeichnet, dass** es sich bei den chemischen Verbindungen um einzelne Moleküle oder Aggregate handelt.

6. Verfahren nach Anspruch 4, **dadurch gekennzeichnet, dass** es sich bei den chemischen Verbindungen um kleine organische Verbindungen, Oligomere oder Polymere handelt.

7. Verfahren nach Anspruch 4, **dadurch** gekenzeichnet, dass es sich bei den chemischen Verbindungen um eine biologisch aktive Substanz und/oder einen pharmazeutischen Wirkstoff handelt.

8. Verfahren nach Anspruch 4, **dadurch gekennzeichnet, dass** es sich bei dem biologischen Material um Nukleotide, Oligonukleotide, Zucker, Lipide, Membranen, Zellen, Zellbestandteile, DNA, RNA, Peptide, Proteine, Antikörper, Haptene oder Antigene handelt.

## Claims

1. A method for targeted labelling of a material to be studied by means of affinity-labelled detection markers, said method comprising the following steps:
a) providing a sample comprising a material to be studied;
b) reacting the material to be studied with a measurable detection marker containing an affinity marker, whereby a labelled material to be studied is formed which, as a mixture, has zero, one or a plurality of detection markers; and
c) separating the material to be studied into non-labelled, singly labelled or multiply labelled fractions by affinity chromatography.

2. The method according to claim 1, **characterized in that** radioactive detection markers or dye markers are used as detection markers.

3. The method according to claim 2, **characterized in that** the dye markers are fluorescence markers.

4. The method according to one of claims 1 to 3, **characterized in that** the material to be studied is
i) a biological material,
ii) a chemical compound,
iii) a synthetic or biological micro-particle or
iv) a combination thereof.

5. The method according to claim 4, **characterized in that** the chemical compounds are single molecules or aggregates.

6. The method according to claim 4, **characterized in that** the chemical compounds are small organic compounds, oligomers or polymers.

7. The method according to claim 4, **characterized in that** the chemical compounds are a biologically active substance and/or a pharmaceutical active ingredient.

8. The method according to claim 4, **characterized in that** the biological material is nucleotides, oligonucleotides, sugars, lipids, membranes, cells, cell constituents, DNA, RNA, peptides, proteins, antibodies, haptens or antigens.

## Revendications

1. Procédé de marquage sélectif de la substance à analyser à l'aide de réactifs de détection contenant un marquage d'affinité, comprenant les étapes consistant à :
a) mettre à disposition un échantillon, comprenant une substance à analyser ;
b) faire réagir la substance à analyser avec un réactif de détection quantifiable, contenant un marqueur d'affinité, où il se forme une substance à analyser marquée, qui ne présente, sous forme de mélange, aucun réactif de détection, ou présente un ou plusieurs réactifs de détection ; et
c) séparer la substance à analyser par chromatographie d'affinité en fractions non marquées, marquées une fois ou marquées plusieurs fois.

2. Procédé selon la revendication 1, **caractérisé en ce que** l'on met en oeuvre comme réactifs de détection des marqueurs de détection radioactifs ou des marqueurs colorés.

3. Procédé selon la revendication 2, **caractérisé en ce que** les marqueurs colorés sont des marqueurs fluorescents.

4. Procédé selon l'une des revendications 1 à 3, **caractérisé en ce que** la substance à analyser est
i) une substance biologique,
ii) un composé chimique,
iii) une microparticule synthétique ou biologique ou
iv) une combinaison de ceux-ci.

5. Procédé selon la revendication 4, **caractérisé en ce que** les composés chimiques sont des molécules isolées ou des groupes de molécules.

6. Procédé selon la revendication 4, **caractérisé en ce que** les composés chimiques sont de petits composés organiques, des oligomères ou des polymères.

7. Procédé selon la revendication 4, **caractérisé en ce que** les composés chimiques sont une substance biologiquement active et/ou un principe actif pharmaceutique.

8. Procédé selon la revendication 4, **caractérisé en ce que** la substance biologique est constituée par des nucléotides, des oligonucléotides, des sucres, des lipides, des membranes, des cellules, des composants cellulaires, de l'ADN, de l'ARN, des peptides, des protéines, des anticorps, des haptènes ou des antigènes.
